Europäisches Patentamt

European Patent Office

Office européen des brevets

(19)

(11) Publication number: **0 159 603 B1**

# (12) EUROPEAN PATENT SPECIFICATION

(45) Date of publication of patent specification: **13.11.91**  (51) Int. Cl.⁵: **G01N 1/28**, G01N 33/50

(21) Application number: **85104178.0**

(22) Date of filing: **05.04.85**

(54) Immunocytochemical microscope control slides.

(30) Priority: **23.04.84 US 602867**

(43) Date of publication of application:
    **30.10.85 Bulletin 85/44**

(45) Publication of the grant of the patent:
    **13.11.91 Bulletin 91/46**

(84) Designated Contracting States:
    **AT BE CH DE FR GB IT LI LU NL SE**

(56) References cited:

    **HISTOCHEMISTRY, vol. 77, no. 2, 1983, pages
    275-279, Springer-Verlag; W.M. HUANG et al.:
    "Improved section adhesion for im-
    munocytochemistry using high molecular
    weight polymers of L-lysine as a slide coat-
    ing"**

    **ACTA CYTOLOGICA, vol. 22, no. 1, 1978,
    pages 15-21, International Academy of Cytol-
    ogy, US; O.A.N. HUSAIN et al.: "A sample
    preparation for automated cervical cancer
    screening"**

    **CANCER GENETICS AND CYTOGENETICS,
    vol. 4, no. 2, 1981, Elsevier North-Holland
    Inc., NL; I.D. DUBE et al.: "A method for**

    **obtaining high quality chromosome prepara-
    tions from single hemopoietic colonies on a
    routine basis"**

(73) Proprietor: **ABBOTT LABORATORIES
    14th Street and Sheridan Road North St
    North Chicago, Illinois 60064(US)**

(72) Inventor: **Konrath, John
    510 Lakehurst Drive
    Waukegan Illinois 60085(US)**
    Inventor: **Miller, Larry S.
    2632 Vermont Avenue
    Waukegan Illinois 60087(US)**

(74) Representative: **Modiano, Guido et al
    MODIANO, JOSIF, PISANTY & STAUB
    Modiano & Associati Via Meravigli, 16
    I-20123 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the preparation of cell and tissue specimens on microscope slides, and more particularly, to the preparation of specimens on immunocytochemical microscope control slides.

Immunocytochemical techniques are used to visualize the presence and distribution of a wide variety of cell and tissue antigens on microscope slides, both in basic research and clinical diagnosis. There are numerous applications in which such slide preparation techniques have been used. To name a few, immunocytochemical techniques have been used for localizing viral antigens during different stages of infection, immunoglobulin synthesis by plasma cells, and receptor localization during endocytosis.

Control slides are utilized in immunocytochemical procedures to provide known positive specimens containing an antigen of interest, or known negative specimens lacking the antigen of interest. The control slides comprise cells, tissue sections or other pathological or cytological preparations which are attached to microscope slides and stored until needed in an immunocytochemical assay. The immunoreactivity, i.e., the ability of antigens in the specimens to combine with antibodies specific for the antigens, and cell morphology of the specimens on the control slides, must be preserved.

Currently, the most commonly used immunocytochemical control slides are made by fixing a specimen such as tissue in formaldehyde, embedding the fixed tissue specimen in paraffin, cutting a tissue section and attaching the section onto a slide. When the specimen is needed for use in an immunocytochemical assay, the paraffin must be removed before use of the slide through oven heating, treatment with xylene and then rehydration of the specimen in a sequence of graded alcohols. This technique is disadvantageous for the reason that immunoreactivity may be lost by reason of the harsh conditions to which specimens are subjected during fixing, paraffin embedding and dewaxing. In addition, such techniques require tissue sections which must be cut one at a time with a microtome, and these tissue sections may vary from one section to another in terms of antigen content and distribution.

The present invention is a method for the preparation of immunocytochemical control slides in which cell lines or tissue sections are adhered to microscope slides which have been treated with polylysine. The slides are then incubated in a humidified chamber without drying the cells. Thereafter, the specimen is fixed, and a water-soluble material, such as a polyalkylene glycol, is applied. The method of the present invention thus permits immunocytochemical control slides to be stored for extended periods of time while preserving immunoreactivity and cell morphology. Since the method of the present invention employs a water-soluble material, it is unnecessary to employ the harsh conditions that are usually employed when removing paraffin from immunocytochemical control slides of the prior art. In contrast, the inventive control slides can simply be immersed in an aqueous bath to remove the water-soluble material thereon and to rehydrate the specimen.

In the preferred practice of the invention, a microscope slide, preferably a glass slide, is first treated with a polylysine solution. It is preferred that the polylysine employed have a molecular weight of 4,000 or greater. Examples of suitable polylysine solutions include an approximately .005-.02% solution of poly-L-lysine hydrobromide ($\geq$ 4,000 MW) or poly-D-lysine hydrobromide ($\geq$ 4,000 MW) in distilled water. The slide is washed with distilled water to remove any excess polylysine. Next, the desired cell line or tissue section is applied to the polylysine-treated slide.

If a cell line is the specimen of choice, the cells are grown in an appropriate culture by a variety of conventional techniques known to those skilled in the art. A suspension of the cells is dropped onto the polylysine-treated slide, and the slide is incubated at an appropriate temperature for a time sufficient to adhere the cells to the polylysine-treated slide. The incubation period should be carried out in a humidified chamber so that the cells are not permitted to dry, to insure that cell immunoreactivity and morphology are preserved.

If a tissue section is used, the tissue section is cut to an appropriate thickness and applied directly to the polylysine-treated slide.

The polylysine serves to secure cells or tissue to the slide because polylysine, which is a cationic polymerized amino acid, attaches to the negatively-charged glass surface, leaving excess positive charge available for the attraction of negatively-charged cells. As a result, the cells remain discrete on the slide and form a fairly well-dispersed monolayer. See, for example, Husain et al., J. Clin. Pathol. 33, 309-311 (1980) and Mazia, et al. J. Cell. Biol. 66, 198-200 (1975).

After the specimen has been adhered on the slide, it is then fixed on the slide by treatment with an appropriate aldehyde-based fixative. The preferred fixative is picric acid paraformaldehyde, known as Zamboni's fixative. Thereafter the slide is washed with a buffered saline solution and then treated with methanol and then with acetone. Preferably, the methanol and acetone are applied at cold temperatures in the range of -15 to -20° C.

Next, the slide is treated with a water-soluble material and preferably a polyethylene glycol. As a general rule, a relatively high molecular weight polyethylene glycol is utilized to preserve the immunoreactivity and cell morphology. For best results, a polyethylene glycol having a molecular weight of at least 4,000 should be used. As will be appreciated by those skilled in the art, other similar water-soluble polymers can likewise be used including, without limitation, other polyalkylene glycols such as polypropylene glycol, polybutylene glycol and the like.

After treatment with the polyethylene glycol, the slides containing the specimens thereon can be stored for extended periods of time. When it is desired to utilize the control slides, such as in an immunocytochemical assay, the water-soluble material is removed, and the underlying specimen is rehydrated by contacting the control slide with an aqueous solution, and preferably a buffered solution, such as a buffered saline, a phosphate buffer, or a tris buffer.

As will be appreciated by those skilled in the art, the concepts of the present invention can be employed in a variety of research or diagnostic applications, and generally in all those applications in which immunocytochemical microscopic slide techniques are used to detect antigens in a specimen. The concepts of the present invention can be used with cell culture specimens as well as tissue sections and other pathological or cytological preparations.

One specific field of application to which the concepts of the present invention are particularly well suited is in the estrogen receptor immunocytochemical technique employed to visualize distribution of estrogen receptor antigen in breast cancer tumors. Thus, estrogen receptor-rich cells can be processed in accordance with the process of the present invention to provide estrogen receptor control slides which can be easily produced and stored intact, dry and at ambient temperatures, while preserving estrogen receptor immunoreactivity and cell morphology.

Having described the basic concepts of the present invention, reference is now made to the following examples, which are provided by way of illustration, and not by way of limitation, of the practice of the present invention in the preparation of control slides and their use in immunocytochemical procedures.

Example 1

This example illustrates the preparation of immunocytochemical control slides using a cell line as the specimen of choice.

A desired cell line is grown in appropriate media and under suitable conditions by conventional culture techniques, in flasks, roller bottles, or other containers. Ninety percent of the culture media is removed, and the remaining cells covered with culture media are harvested from the inside surface of the container using any suitable method such as gentle scraping with a rubber policeman or by rinsing with trypsin or EDTA. A suspension of cells is then transferred to a suitable container.

One drop of the cell suspension is dropped onto each polylysine-treated microscope slide. The polylysine-treated slides are prepared by placing clean microscope slides in a solution of poly-L-lysine hydrobromide (≥4,000 MW) or poly-D-lysine hydrobromide (≥ 4,000 MW) in distilled water at a dilution of one hundred micrograms per milliliter distilled water. The polylysine-treated slides are placed in distilled water for one minute, and this rinse is repeated once. The slides are then air-dried. For best results, the slides should be made fresh daily.

After the cell suspension is dropped onto the polylysine-treated slides, the slides are incubated for fifteen minutes at 37°C in a humidified chamber. The cells should not be allowed to dry.

The cells are then fixed onto the microscope slide by contacting the slide with the following solutions: (1) Zamboni's fixative for 35 minutes; (2) phosphate buffered saline (.01 M, pH 7.4) for five minutes; repeat; (3) methanol (absolute) at -20°C for four minutes; and (4) acetone at -20°C for one minute.

Next, the slides are treated for five minutes with a solution of polyethylene glycol (≥ 4,000 MW) containing approximately six grams of polyethylene glycol in 100 ml. distilled water. The control slides are then allowed to air dry and can be stored at ambient or other suitable temperatures until needed.

Example 2

This example illustrates the preparation of immunocytochemical control slides using frozen tissue sections as the specimen of choice.

Frozen tissue sections having a thickness of 4 to 6 μm are cut from the desired tissue using a cryostat. Without drying the sections, each section is transferred to a slide which has been coated with polylysine as described in Example 1. The tissue section is then fixed and overcoated using the same technique as described in Example 1, by treating with Zamboni's fixative, a phosphate-buffered saline solution, methanol, acetone and finally with polyethylene glycol.

Example 3

This example illustrates the preparation of an estrogen receptor control slide.

A two-week old roller bottle culture of estrogen receptor rich cells is processed to remove all but 50 ml of the culture media. The cells attached to the inside surface of the roller bottle and covered with media, are gently scraped with a rubber policeman and transferred to centrifuge tubes. One drop of the suspended cells is placed onto each etched circle of a microscope slide treated with polylysine as described in

Example 1.

After incubation for fifteen minutes at 37°C in a humidified chamber as described in Example 1, the cells are fixed to a microscope slide and treated with polyethylene glycol, using the same procedure described in Example 1. These slides serve as controls for estrogen receptor immunocytochemical assays, and can be stored intact, dry and at ambient temperature while preserving estrogen receptor immunoreactivity and cell morphology.

As will be appreciated by those skilled in the art, the concepts of the present invention provide immunocytochemical control slides containing a specimen which is protected from the environment by a water-soluble material such as polyethylene glycol. The polyethylene glycol can be removed and the specimen rehydrated for use by simply immersing or otherwise treating the slide with, for example, a phosphate-buffered saline solution.

The immunocytochemical control slides of the present invention have many advantages over prior formalin-fixed, paraffin-embedded tissue sections. First, the inventive control slides are fixed mildly, do not require paraffin embedding and require only quick, mild dewaxing before use. This enables immunoreactivity and cell morphology to be preserved. Second, the inventive slides enable the use of cultured cell lines containing an antigen of interest, which provides a consistent and homogenous source of antigen so that a large number of control slides can be prepared from a single culture vessel of cells. This is important in the production of a standardized immunocytochemical assay in order to provide positive and negative control slides that do not vary from batch to batch. Finally, the method of the present invention is quick, simple and readily usable in the preparation of large numbers of stable control slides.

## Claims

1. A method for the preparation of immunocytochemical slides comprising:
   (a) treating said slides with a polylysine solution.
   (b) adhering cell or tissue specimens on said polylysine-treated slides;
   (c) treating said cell or tissue specimens on said slides with a fixative; and
   (d) treating said slides with a water-soluble polyalkylene glycol.

2. The method of Claim 1 wherein said fixative comprises an aldehyde-based fixative, phosphate-buffered saline, methanol and acetone.

3. The method of Claim 2 wherein said methanol and said acetone are applied to said slide at a temperature of -20°C.

4. The method of Claim 1 wherein said polyalkylene glycol is polyethylene glycol.

5. The method of Claim 4 wherein said polyethylene glycol has a molecular weight of at least 4,000.

6. A control slide for use in an immunocytochemical assay comprising a polylysine-treated slide having a cell or tissue specimen fixed thereon and having a water-soluble polyalkylene glycol overcoating.

7. The control slide of Claim 6 wherein said cell or tissue specimen is fixed on said slide by contacting said slide with Zamboni's fixative, phosphate-buffered saline, methanol and acetone.

8. The control slide of Claim 6 wherein said polyalkylene glycol is polyethylene glycol.

## Revendications

1. Procédé de préparation de lames immunocytochimiques comprenant :
   (a) le traitement desdites lames par une solution de polylysine ;
   (b) le collage des pièces cellulaires ou tissulaires sur lesdites lames traitées par la polylysine ;
   (c) le traitement desdites pièces cellulaires ou tissulaires sur lesdites lames à l'aide d'un fixateur ; et
   (d) le traitement desdites lames à l'aide d'un polyalkylèneglycol hydrosoluble.

2. Procédé selon la revendication 1, dans lequel ledit fixateur comprend un fixateur à base d'aldéhyde, une solution salée tamponnée au phosphate, du méthanol et de l'acétone.

3. Procédé selon la revendication 2, dans lequel ledit méthanol et ladite acétone sont appliqués à ladite lame à une température de -20°C.

4. Procédé selon la revendication 1, dans lequel ledit polyalkylèneglycol est du polyéthylèneglycol.

5. Procédé selon la revendication 4, dans lequel ledit polyéthylèneglycol a un poids moléculaire d'au moins 4 000.

6. Lame de contrôle pour usage dans un dosage immunocytochimique, comprenant une lame traitée par la polylysine sur laquelle une pièce cellulaire ou tissulaire est fixée et recouverte de polyalkylèneglycol hydrosoluble.

7. Lame de contrôle selon la revendication 6, dans laquelle ladite pièce cellulaire ou tissulaire est fixée sur ladite lame par mise en contact de ladite lame avec le fixateur de Zamboni, une solution salée tamponnée au phosphate, du méthanol et de l'acétone.

8. Lame de contrôle selon la revendication 6, dans laquelle ledit polyalkylèneglycol est du polyéthylèneglycol.

**Patentansprüche**

1. Verfahren zur Herstellung von immunzytochemischen Objektträgern, welches Verfahren umfaßt:

(a) Behandeln der Objektträger mit einer Polylysinlösung;

(b) Befestigen von Zell- oder Gewebeproben an den dem Polylysin behandelten Objektträgern;

(c) Behandeln der Zell- oder Gewebeproben auf den Objektträgern mit einem Fixiermittel; und

(d) Behandeln der Objektträger mit einem wasserlöslichen Polyalkylenglykol.

2. Verfahren nach Anspruch 1, wobei das Fixiermittel ein Fixiermittel auf Aldehydbasis, Phosphat-gepufferte Kochsalzlösung, Methanol und Aceton umfaßt.

3. Verfahren nach Anspruch 2, wobei das Methanol und das Aceton auf den Objektträger bei einer Temperatur von -20° C aufgetragen werden.

4. Verfahren nach Anspruch 1, wobei das Polyalkylenglykol Polyethylenglykol ist.

5. Verfahren nach Anspruch 4, wobei das Polyethylenglykol ein Molekulargewicht von wenigstens 4000 hat.

6. Kontroll-Objektträger zur Verwendung in einem immunzytochemischen Test, welcher Objektträger einen mit Polylysin behandelten Objektträger umfaßt, auf welchem eine Zell- oder Gewebeprobe fixiert worden ist, und der eine wasserlösliche Deckschicht aus Polyalkylenglykol aufweist.

7. Kontroll-Objektträger nach Anspruch 6, wobei die Zell- oder Gewebeprobe auf dem Objektträger dadurch fixiert worden ist, daß man diesen Objektträger mit Zambonis Fixiermittel, mit Phosphat-gepufferter Kochsalzlösung, mit Methanol und Aceton in Berührung gebracht hat.

8. Kontroll-Objektträger nach Anspruch 6, wobei das Polyalkylenglykol Polyethylenglykol ist.